# EUROPEAN PATENT APPLICATION

(11) **EP 2 591 719 A2**
(43) Date of publication of application: **15.05.2013**
(21) Application number: 11803773.8
(22) Date of filing: 05.07.2011
(51) Int. Cl.: A61B 5/022, A61B 5/02

(54) **CUFF FOR A BLOOD PRESSURE METER**

(30) Priority: 05.07.2010 KR 20100064427
(71) Applicant: Park, Jong-Eun, Seoul 135-500 (KR); Cha, Jae-Soon, Seoul 135-240 (KR)
(72) Inventor: Park, Jong-Eun, Seoul 135-500 (KR); Cha, Jae-Soon, Seoul 135-240 (KR)
(74) Representative: Kudlek & Grunert Patentanwälte
(86) International application number: PCT/KR2011/004897
(87) International publication number: WO 2012/005487

(57) **Abstract**

The present invention relates to a cuff for a blood pressure meter, and more particularly relates to a cuff for a blood pressure meter, which is jointed to a blood pressure for measuring blood pressure and is worn on the body in such a way as to compress a blood pressure measurement site, and which comprises: a cuff band which is worn around the blood pressure measurement site of the body and is provided with an expansion bladder that expands on the inside; a fluid supply apparatus which is connected to the cuff band and supplies a fluid so as to make the expansion bladder expand; and a bladder separator which is provided in the cuff band and splits the expansion bladder, that is expanded by means of the fluid supply apparatus, into at least two or more pats so as to effect split expansion of the same. When the present invention is employed, because the expansion bladder which compresses the blood pressure measurement site is split by means of the ladder separator, there is the advantageous effect that it expands and contracts to a length and width that match the firth of the blood pressure measurement site and accurate blood pressure measurement can be taken.

## Description

### Technical Field

The present invention relates to a cuff for a blood pressure meter and more particularly to a cuff for a blood pressure meter which is worn on a blood pressure measurement portion so as to measure a blood pressure for thereby pressurizing the blood pressure measurement portion.

### Background Art

Since 1905 when Korotkoff developed an auscultation for the purpose of indirectly measuring a blood pressure based on a Korotkoff sound, the indirect blood pressure measurement still remains unchanged in whole for about 100 years.

The Korotkoff method (auscultation method) has features in that like an oscillometric method the cuff pressure is once raised above a systolic blood pressure, and the blood flow is stopped, and then the cuff pressure is forced to slowly drop. The Korotkoff sound generating at the timing when the once stopped blood pressure resumes is detected at an end portion corresponding to the downstream of the cuff, and the internal pressure of the cuff for a blood pressure measurement is obtained as a blood pressure value of a systolic blood pressure (highest blood pressure), and the internal pressure of the cuff at the time the Korotkoff sound disappears is obtained as a blood pressure value of a diastolic blood pressure (lowest blood pressure).

Sine the introduction of the theory that was designed to measure the blood pressure with cuff-oscillometer in 1965, the theory and technology have been developed, so the identification method for the systolic and diastolic blood pressures is established. The above mentioned cuff-oscillometer method has features in that the cuff pressure is once raised above the systolic blood pressure, and the vibrations of the artery generating based on the changes in the volumes of the artery when the cuff pressure slowly drops are detected, thus determining the blood pressure with the aid of the changes in the amplitudes of the vibrations.

The cuff-oscillometer method is directed to obtaining the phenomenon that the blood flow resumes, from the changes in the pressure appearing in the overlapping form in the cuff pressure generating due to the changes in the volume of the artery below the cuff. A microphone or a stethoscope for the sake of detecting Korotkoff sounds is not necessary. Thanks to the advantages that the number of necessary parts is smaller and the manufacture does not cost a lot as compared to the Korotkoff method, most of the electronic type automated blood pressure meters is made using the cuff-oscillometer.

The criteria of the blood pressure measurement method in terms of clinic and research is the temporal blood pressure at the doctor's examining room; however there is a certain difference between the blood pressure measured at the doctor's examining room and the blood pressure measured outside by a patient himself since the introduction of the blood pressure self-measurement method in 1940. In other words, there might be a big difference in terms of the quality and quantity in the blood pressure information depending on the blood pressure measurement methods.

In recent years, it is proved that the blood pressure during the ambulatory blood pressure and the home blood pressure have more important clinic values as compared to the temporal blood pressure in the doctor's examining room because the ambulatory blood pressure and the home blood pressure appear to have more frequencies in the measurements within unit time, so the problems relative with the quantity and quality of the information on the blood pressure occur.

The electronic blood pressure meter is classified into a forearm type, a wrist type and a finger type. The method of measuring the blood pressure of a finger is easy and convenient to use; however the blood pressure of a finger is measured different from the blood pressure of the forearm in terms of a biological view and has a convulsion in the winter, and a difference inevitably occurs in the hydrostatic pressure.

The wrist-worn blood pressure meter is easy to carry and is convenient to use; however it has a difference in terms of the hydrostatic pressure.

The standard position for measuring a blood pressure measurement is the right atrium. The portion of the measurement of the blood pressure is positioned 10cm below from the right atrium being measured 7mmHg higher than the forearm (portion of the right atrium) in both the systolic and diastolic blood pressures, and when the portion of the measurement of the blood pressure is positioned 10cm above from the right atrium, the blood pressure is measured 7mmHg lower. So, the wrist blood pressure meter is recommended to be used with the wrist being positioned at the height of the heart; however such posture is not generally obtained, which results in a nonuniform blood pressure measurement.

The forearm blood pressure meter is configured to measure the portion of the forearm where the right atrium is positioned, the features of which make sure that a more accurate blood pressure can be measured as compared to the finger type or the wrist type; however the above mentioned hydrostatic pressure problem should be cared, and the forearm blood pressure meter is recommended for a person having a thick forearm or a thin forearm to use a large size cuff or a small size cuff.

In 1993 the US heart association recommended for people to use the optimized width and length of the expansion portion pressurizing the forearm as it expands in injecting air into the cuff. The recommended width of the expansion portion is 40% of the surrounding dimension at the intermediate portion of the forearm, and the length is 80% of the surrounding dimension at the intermediate portion of the forearm.

In details, the US heart association regulates that in case of a child with 16-21cm of the surrounding of the forearm, the width of the expansion portion is 6cm, and the length is 21cm, and in case of an ordinary adult with 27-34cm of the surrounding of the forearm, the width of the expansion portion is 13cm, and the length is 30cm, and in case of a large adult with 35-44cm of the forearm, the width of the expansion portion is 16cm, and the length is 38cm, and in case of an adult with 45-52cm of the very thick surrounding of the forearm, the width of the expansion portion is 20cm, and the length is 42cm.

According to the 1999-2002 health and nutrition evaluations in the USA published in 2005, the results in changes of the surroundings of the forearms of the USA people show that the values are different depending on the sex, age, race and peoples, and the values appear to increase depending on the diseases such as hypertension or diabetics; however there are meaningful increases in all ranges of ages.

In case of Korea, about 30% of the total populations appear to have obesity according to the national nutrition evaluation in 2008, and it is regret that there are not any information on the surrounding length of the arms, but a little increase might be assumed.

In conclusion, it is problematic that the blood pressure measured using a cuff having a uniformly sized expansion portion might be higher or lower than an actual blood pressure because the blood pressure is measured without reflecting the physical features.

The automated measurement apparatus using an oscillometric method is semi-permanently used at home in consideration of the importance of a home blood pressure because the blood pressure is one of the best accuracy and reliability ways in estimating the symptoms of the side effects in terms of the heart and blood vessel-related diseases. The user who measures blood pressures without considering the physical features might provide a wrong blood pressure to a doctor who is in charge of the patient. The wrongly measured blood pressure values might cause as much as problems in managing the intakes of the blood pressure medicines.

### Disclosure of Invention

Accordingly, the present invention is made to improve the problems encountered in the conventional art. It is an object of the present invention to provide a cuff for a blood pressure meter which makes it possible to accurately measure a blood pressure in such a way that an expansion portion pressurizing a forearm is split and expanded and flexibly adjusted in conformity to the surrounding length of the forearm.

It is another object of the present invention to provide a cuff for a blood pressure meter having features in that the length and width of the expansion portion expanding by fluid are split, and the fluid is injected by way of a member intermitting the fluid with the aid of the split expansion portions.

It is further another object of the present invention to provide a cuff for a blood pressure meter having features in that an expansion portion is split into a basic space matching with a thin forearm like a child and an additional expansion space matching with a thick forearm like an adult and expands.

It is still further another object of the present invention to provide a cuff for a blood pressure meter which has features in that the surrounding length of a forearm is detected, and an expansion portion is expanded and contracted in conformity with the surrounding length of the detected forearm.

It is still further another object of the present invention to provide a cuff for a blood pressure meter which has a member configured to measure the surrounding thickness of a forearm.

To achieve the above objects, there is provided a cuff for a blood pressure meter which is engaged to a blood pressure meter for the sake of the measurement of blood pressure and is worn on a human body for thereby pressurizing a measurement portion of blood pressure, comprising a cuff band which is worn on a measurement portion of a blood pressure of a human body and has an expansion bladder in the cuff band, the expansion bladder being configured to expand; a fluid supplier which is connected with the cuff band and allows the expansion bladder to expand by supplying fluid; and a bladder separator which splits the expansion bladder into at least two parts for the sake of split step-by-step expansions.

The bladder separator comprises a splitting wall which is provided at the expansion bladder and splits the expansion space of the expansion bladder expanding by means of the fluid supplier into at least two parts; and an expansion valve which is provided at the splitting wall and intermits the fluid supplied from the fluid supplier.

The splitting wall comprises a main wall which defines the expansion space of the expansion bladder as a basic expansion space pressurizing the measurement portion of the blood pressure of a child; and at least one extension wall which is provided at one side of the main wall and defines an additional expansion space at a side portion of the basic expansion space.

In addition, the bladder separator comprises a thickness measurement sensor which is provided at one side of the expansion bladder and serves to measure the thickness of the measurement portion of the blood pressure; and a controller which serves to open and close the expansion valve in conformity with the thickness of the measurement portion of the blood pressure measured by the thickness measurement sensor.

In addition, there is further provided a thickness measurement member which is provided at the cuff band and serves to measure the thickness of the measurement portion of the blood pressure on which the cuff band is worn, the thickness measurement member being formed of one selected between a tape measure and a thickness detection sensor which is attached to one side of the cuff band.

### Advantageous effects

According to the cuff for a blood pressure meter of the present invention, an expansion bladder pressurizing a measurement portion of a blood pressure is split by means of a bladder separator, and it is expanded with the length and width matching with the thickness of the measurement portion of the blood pressure, thus measuring an accurate blood pressure.

In addition, the bladder separator is split into at least two splitting walls and is communicated by way of an expansion valve which intermits fluid, so the expansion bladder can be expanded in conformity with the thickness of the measurement portion of a blood pressure.

In addition, the expansion bladder helps measure an accurate blood pressure with the aid of a basic expansion space formed by a main wall for the sake of a child who has a thin forearm and an additional expansion space formed by an extension wall for the sake of an adult who has a thick forearm.

The expansion bladder is configured to detect the thickness of the measurement portion of a blood pressure by means of a thickness measurement sensor, and it is expanded in conformity with the thickness detected as the expansion valve is adjusted by a controller. So, the present invention can be well applied to an electronic blood pressure meter or an automated blood pressure device.

Since the thickness of a forearm can be measured by a tape measure which is a thickness measurement member provided at a cuff band or a thickness detection sensor, the basic expansion space and the additional expansion space can be expanded by adjusting the expansion valve in conformity with the measured thickness.

### Brief Description of Drawings

Figure 1 is a front view illustrating a cuff for a blood pressure meter according to an embodiment of the present invention.

Figure 2 is a front view illustrating an expansion bladder of a cuff for a blood pressure meter of Figure 1.

Figure 3 is a vertical cross sectional view illustrating an operation of an expansion bladder of Figure 2.

Figure 4 is a view illustrating a state that a cuff for a blood pressure meter of Figure 1 is installed at a conventional automated blood pressure measurement device.

### Best Modes for carrying out the invention

The preferred embodiments of the present invention will be described in details with reference to the accompanying drawings, and during the descriptions of the present invention, the same elements and functions will be given the same reference numerals.

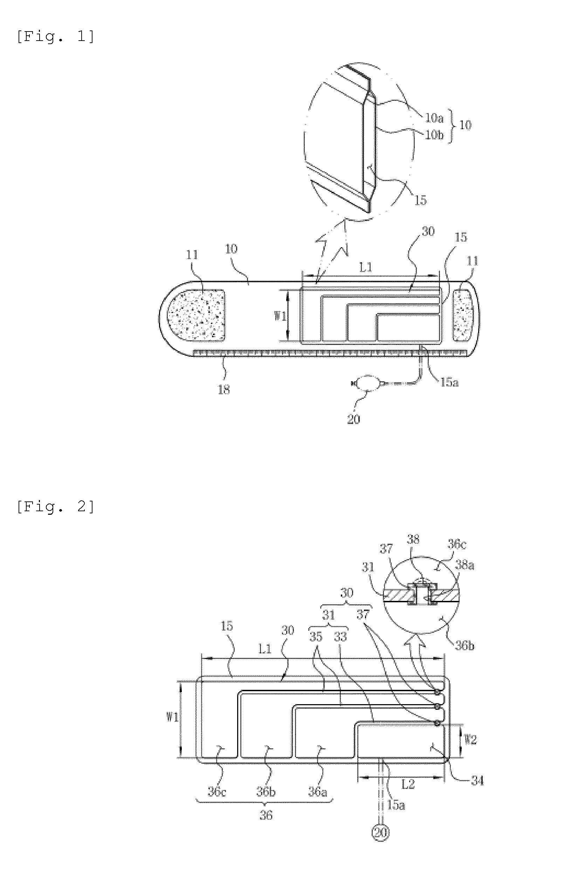

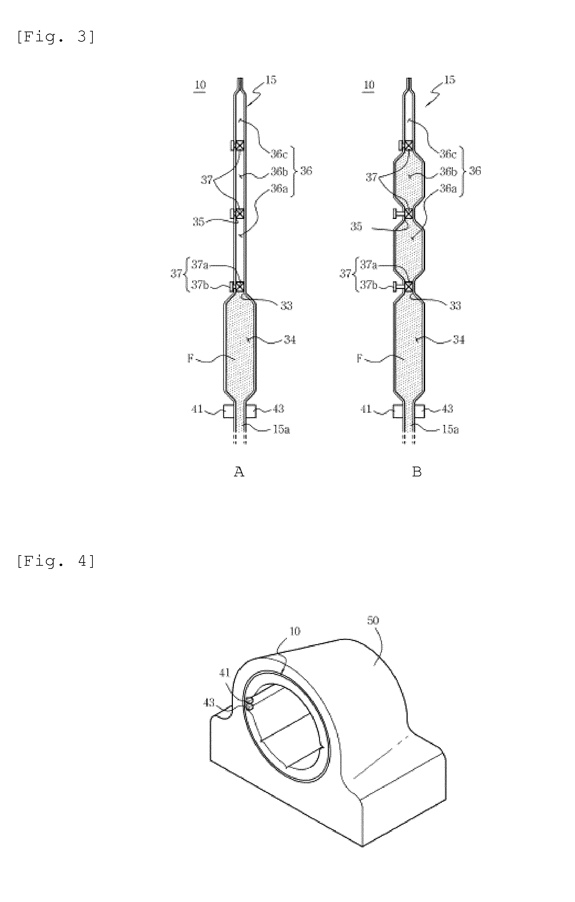

Figure 1 is a front view illustrating a cuff for a blood pressure meter according to an embodiment of the present invention. Figure 2 is a front view illustrating an expansion bladder of a cuff for a blood pressure meter of Figure 1. Figure 3 is a vertical cross sectional view illustrating an operation of an expansion bladder of Figure 2.

As shown in Figure 1, the cuff for a blood pressure meter according to the present invention comprises a cuff band 10, a fluid supplier 20 and a bladder separator 30.

The cuff band 10 is worn on a measurement portion of a blood pressure of a body during the measurement of a blood pressure, and an expansion bladder 15 is provided in the interior of the cuff band 10. It is preferred that the cuff bad 10 is made from a flexible material.

The cuff band 10 is formed of two overlapping sheets 10a and 10b, and the rim portions of the overlapped sheets 10 are bonded with each other or they can be formed by way of a bonding member sealing the rim portions of the sheets 10a and 10b.

Provided at both sides of the cuff band 10 are a fastener 11, respectively, and the fasteners 11 are engaged with each other when wearing the cuff band 10 for thereby fixing the cuff band 10 at the measurement portion of a blood pressure.

Here, it is preferred that the measurement portion of a blood pressure on which the cuff band 10 is worn is a forearm (an arm portion above the elbow joint) at the same height as the right atrium of the heart.

The expansion bladder 15 is provided at the inner side of the cuff band 10 and is worn on the measurement portion of the blood pressure along with the cuff band 10 and is expanded by means of the fluid supplied from the fluid supplier 20 for thereby pressurizing the measurement portion of the blood pressure. The expansion bladder 15 is a space formed by the overlapping sheets 10a and 10b as shown in the drawings.

The expansion bladder 15 is preferably formed with a set length L1 and a set width W1. The set length L1 and width W1 are the maximum length and width long enough to worn on the measurement portion of the blood pressure. For example, the expansion bladder 15 has a length L1 of 42cm and a width W1 of 20cm long enough to be worn on the thick forearm of an adult.

The fluid supplier 20 is configured to supply fluid such as gas or liquid to the expansion bladder 15 as it is connected with the cuff band 10. The fluid supplier 20 is connected with the expansion bladder 15 by way of an inlet 15a formed at the expansion bladder 15 and as shown in the drawing it might be formed of a conventional manual pump serving to supply fluid with the aid of a physical strength. The fluid supplier 20 might be formed of a conventional automated pump configured to work by means of electric power for the purpose of supplying fluid. So, the expansion bladder 15 can be expanded by means of the fluid of the fluid supplier 20.

The expansion bladder 15 is connected with a blood pressure meter serving to measure the blood pressure by measuring the expansion pressure.

The cuff for a blood pressure meter according to the present invention further comprises a thickness measurement member 18. The thickness measurement member 18 might be a tape measure attached to the cuff band 10 and worn on the measurement portion of the blood pressure for thereby measuring the thickness of the measurement portion of the blood pressure. The thickness measurement member 18 might be formed of a thickness measurement sensor which can detect the thickness of the measurement portion of the blood pressure as it is attached to the cuff band 10.

The bladder separator 30 is provided at the expansion bladder 15 and splits the expansion bladders 15 expanding by means of the fluid supplier 20 into at least two parts for thereby performing a split expansion.

As shown in Figure 2, the bladder separator 30 comprises a splitting wall 31 provided at an inner side of the expansion bladder 15 and an expansion valve 37.

The splitting wall 31 is formed as the sheets 10a and 10b are bonded with each other in the interior of the expansion bladder 15. Here, the splitting wall 31 comprises a main wall 33 and an expansion wall 35.

The main wall 33 forms the basic expansion space 34 in the interior of the expansion bladder 15. Here, the basic expansion space 34 is connected with the fluid supplier 20 by way of the inlet 15a and has a minimum length L2 and a minimum width W2 long enough to be worn on the measurement portion of the blood pressure.

The basic expansion space 34 has 12cm of a length L2 and 6cm of a width W2 which are long enough to be worn on the thin forearm like a child. So, the expansion bladder 15 serves to form a basic expansion space 34 by means of the main wall 33 for thereby pressurizing the forearm of a child.

The extension wall 35 is provided at one side of the main wall 33 for thereby forming the additional expansion space 36 extending the length and width of the basic expansion space 34. The additional expansion space 36 expands from the front and lateral sides of the basic expansion space 33 for thereby expanding the expansion portion which expands by means of the basic expansion space 34.

As shown in the drawings, the additional expansion space 36 is formed in an upside down shape of " 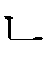 " for thereby expanding the length and width of the expansion portion expanding by the basic expansion space 34.

The extension wall 35 is partitioned into at least one part for thereby forming additional expansion spaces 36a, 36b and 36c. The additional expansion space 36 is split into at least two parts so they can be sequentially expanded. The first additional expansion space 36a has 30cm of a length and 13cm of a width long enough to well fit the forearm of an ordinary adult, and the second additional expansion space 36b has 38cm of a length and 16cm of a width long enough to well fit the thicker forearm of an adult, and the third additional expansion space 36c has 42cm of a length and 20cm of a width long enough to fit the very thick forearm of the adult.

The expansion bladder 15 expands with a minimum length L2 and a minimum width W2 by means of the main wall 33 and expands with a maximum length L1 and a maximum width W1 by means of the extension wall 35.

The expansion valve 37 is provided at the splitting wall 31 for thereby intermitting the fluid supplied from the fluid supplier 20. In other words, the expansion valve 37 is provided at the main wall 33 and the extension wall 35, respectively, and intermits the fluid for thereby expanding the expansion portion of the expansion bladder 15 in conformity with the thickness of the forearm.

As shown in the drawings, the expansion valve 37 is preferably formed at an end portion of the expansion bladder 15. The expansion valve 37 might be formed of a through hole 38a and a cap 38. The cap 38 serves to intermit the fluid and to be curved and open by means of the pressure as indicated by the broken lines in the drawings, and the fluid flows by way of the through hole 38a and the opened cap 38.

For example, the expansion valve 37 might be formed of an air supply valve (check valve) made from a rubber material generally installed at a tube for a water entertainment. The expansion valve 37 might be formed of a conventional air supply value made from a material which has certain flexibility and sealing performance.

As shown in Figure 3, the expansion valve 37 comprises a valve body 37a intermitting fluid and an opening and closing valve 37b adjusting the valve body 37a. The fluid F is introduced by way of the inlet 15a and expands the basic expansion space 34 of the expansion bladder 15 and is intermitted by means of the main wall 33 and the expansion valve 37.

When expanding the additional expansion space 36, the expansion valve 37 supplies the fluid F to the additional expansion space 36 as the valve body 37a is opened by the opening and closing valve 37b. The expansion valve 37 might be formed of a conventional solenoid valve operating by electric power.

The bladder separator 30 might be further formed of a thickness measurement sensor 41 and a controller 43. The thickness measurement sensor 41 is provided at an outer side of the expansion bladder 15 for thereby detecting the thickness of the measurement portion of the blood pressure worn on the expansion bladder 15, and the detected thickness signal is applied to the controller 43.

The thickness measurement sensor 41 serves to automatically detect the thickness of the forearm where the blood pressure will be measured, and the controller 43 allows the valve body 37a of the expansion valve 37 to open and close in accordance with the thickness signal of the forearm detected by the thickness measurement sensor 41. The width and length of the expansion bladder 15 can be adjusted as it is automatically expanded by means of the thickness measurement sensor 41 and the controller 43

As shown in Figure 4, the cuff for a blood pressure meter according to the present invention can be installed at the conventional automated blood pressure measurement device 50. Here, the cuff band 10 is installed inside the automated blood pressure measurement device 50, and the expansion bladder 15 is automatically expanded by the thickness measurement sensor 41 and the controller 43 in conformity with the thickness of the forearm. Since the known automated measurement device is used, the detailed descriptions will be omitted.

The operation of the cuff for a blood pressure meter according to the present invention including the above described elements will be described. The cuff for a blood pressure meter of the present invention is generally worn on the body of the user when measuring the blood pressure.

As shown in Figure 1, the cuff band 10 is worn on the measurement portion of the blood pressure, preferably, on the forearm. At this time, the cuff band 10 is worn on the forearm and is fixed by the fastener 10a provided at both sides.

When the cuff band 10 is worn on the forearm, the thickness of the forearm is measured by the thickness measurement member 18 which is the tape measure or the thickness measurement sensor. The expansion length and the expansion width of the expansion bladder 15 are determined in conformity with the thickness of the forearm.

The expansion bladder 15 is worn on the forearm along with the cuff band 10 and is expanded by the fluid supplier 20 for thereby pressurizing the forearm.

As shown in Figure 2, the bladder separator 30 split-expands the expansion bladder 15 in conformity with the thickness of the forearm.

The expansion bladder 30 is split into the basic expansion space 34 and the additional expansion space 36 by means of the main wall 33 and the extension wall 35 and is made communicated by the expansion valve 37.

When the pressure is measured at the thin forearm like a child, the basic expansion space 34 expands, and the additional expansion space 35 is split to operate at multiple stages for the sake of step-by-step expansions when measuring the blood pressures of the middle forearm adult 36a, the thick forearm adult 36b and the very thick forearm adult 36c.

Therefore, the expansion bladder 15 expands with a minimum length L2 and a minimum width W2 by means of the main wall 33, and it expands step by step by means of the extension wall 35 and expands with a maximum length L1 and a maximum width W1.

As shown in Figure 3, "A" shows a case when the blood pressure of a child is measured, and "B" shows a case when the blood pressure of the adult having a thick forearm is measured.

As shown in "A" of Figure3, a tester closes the valve body 37a by adjusting the opening and closing valve 37b of the expansion valve 37 provided at the main wall 33 in case when the testee is a child, and the fluid F is supplied to the fluid supplier 20 by way of the inlet 15a, so the basic expansion space 34 is expanded for thereby pressurizing the forearm.

As shown in "B" of Figure 3, the tester opens the expansion valve 37 provided at the main wall 33 and the extension wall 35 when the thickness of the forearm of the testee is thicker than the ordinary adult and closes only the expansion valve 37 intermitting the third additional expansion space 36c. At this time, the fluid F allows the basic expansion space 34 to open and is introduced by way of the opened expansion valve 37, so the first and second additional expansion spaces 36a and 36b are expanded, thus pressurizing the forearm.

When the pressurizing is finished, the blood pressure is measured using the blood pressure meter, and the test is completed.

The expansion bladder 15 expands in conformity with the thickness of the forearm by the controller 43 allowing the expansion valve 37 to open and be closed in conformity with the detected thickness as the thickness of the forearm is detected by the thickness measurement sensor 41 for thereby pressurizing the blood pressure measurement portion.

As shown in Figure 4, the cuff band 10 is installed at the automated blood pressure measurement device 50, and the expansion bladder 15 automatically expands by means of the thickness measurement sensor 41 and the controller 43 in conformity with the thickness of the forearm.

As described above, according to the cuff for a blood pressure meter according to the present invention, the expansion bladder pressurizing the blood pressure measurement portion does not expand in only the uniform lengthwise and widthwise directions, but it becomes flexible in conformity with the thickness of the measurement portion of the blood pressure, so the accurate blood pressure can be measured. The cuff for a blood pressure meter of the present invention can be well applied to a manual type blood pressure meter or an electronic type blood pressure meter or an automated blood pressure measurement device.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

### Industrial Applicability

The cuff for a blood pressure meter according to the present invention can be well applied to a manual type blood pressure meter or an electronic type blood pressure meter or an automated blood pressure measurement device for the sake of testee of blood pressure, so the present invention can be adapted to the medical instrument industry field.

## Claims

1. A cuff for a blood pressure meter which is engaged to a blood pressure meter for the sake of the measurement of blood pressure and is worn on a human body for thereby pressurizing a measurement portion of blood pressure, comprising:
a cuff band which is worn on a measurement portion of a blood pressure of a human body and has an expansion bladder in the cuff band, the expansion bladder being configured to expand;
a fluid supplier which is connected with the cuff band and allows the expansion bladder to expand by supplying fluid; and
a bladder separator which splits the expansion bladder into at least two parts for the sake of split step-by-step expansions.

2. The cuff of claim 1, wherein the bladder separator comprises:
a splitting wall which is provided at the expansion bladder and splits the expansion space of the expansion bladder expanding by means of the fluid supplier into at least two parts; and
an expansion valve which is provided at the splitting wall and intermits the fluid supplied from the fluid supplier.

3. The cuff of claim 2, wherein the splitting wall comprises:
a main wall which defines the expansion space of the expansion bladder as a basic expansion space pressurizing the measurement portion of the blood pressure of a child; and
at least one extension wall which is provided at one side of the main wall and defines an additional expansion space at a side portion of the basic expansion space.

4. The cuff of claim 2, wherein the bladder separator comprises:
a thickness measurement sensor which is provided at one side of the expansion bladder and serves to measure the thickness of the measurement portion of the blood pressure; and
a controller which serves to open and close the expansion valve in conformity with the thickness of the measurement portion of the blood pressure measured by the thickness measurement sensor.

5. The cuff of claim 1, further comprising:
a thickness measurement member which is provided at the cuff band and serves to measure the thickness of the measurement portion of the blood pressure on which the cuff band is worn, the thickness measurement member being formed of one selected between a tape measure and a thickness detection sensor which is attached to one side of the cuff band.
